Europäisches Patentamt

(19) European Patent Office

Office européen des brevets

(11) Veröffentlichungsnummer: **0 173 177 B1**

(12) **EUROPÄISCHE PATENTSCHRIFT**

(45) Veröffentlichungstag der Patentschrift: **22.04.92**

(51) Int. Cl.5: **C12N 15/85**, C12N 7/00, //C12R1/91

(21) Anmeldenummer: **85110239.2**

(22) Anmeldetag: **16.08.85**

(54) **Enhancer für eukaryotische Expressionssysteme.**

(30) Priorität: **24.08.84 DE 3431140**

(43) Veröffentlichungstag der Anmeldung:
**05.03.86 Patentblatt 86/10**

(45) Bekanntmachung des Hinweises auf die
Patenterteilung:
**22.04.92 Patentblatt 92/17**

(84) Benannte Vertragsstaaten:
**AT BE CH DE FR GB IT LI LU NL SE**

(56) Entgegenhaltungen:

**PROCEEDINGS OF THE NATIONAL ACADEMY OF SCIENCES OF THE UNITED STATES OF AMERICA, Vol. 81, No. 3, Februar 1985 (Baltimore, USA) D.R. THOMSEN et al. "Promoter-regulatory region of the major immediate early gene of human cytomegalovirus" Seiten 659-663**

**JOURNAL OF VIROLOGY, Vol. 49, No. 2, Februar 1984 (Washington D.C.) G. JAHN et al. "Predominant Immediate-Early Transcripts of Human Cytomegalovirus AD 169" Seiten 363-370**

(73) Patentinhaber: **BEHRINGWERKE Aktiengesellschaft
Postfach 1140
W-3550 Marburg 1(DE)**

(72) Erfinder: **Fleckenstein, Bernhard, Prof. Dr.
Haus-Nr. 93
W-8551 Schlaifhausen(DE)**
Erfinder: **Schaffner, Walter, Prof. Dr.
Bodenstrasse 9
CH-8104 Weiningen(CH)**
Erfinder: **Boshart, Michael, Dr.
Zeppelinstrasse 41
W-6900 Heidelberg(DE)**
Erfinder: **Weber, Frank, Dr.
Engerfeldstrasse 9
CH-4310 Rheinfelden(CH)**
Erfinder: **Jahn, Gerhard, Dr.
Effeltricher Strasse 11
W-8524 Neunkirchen(DE)**
Erfinder: **Dorsch-Häsler, Karoline, Dr.
Apfelbaumstrasse 43
CH-8050 Zürich(CH)**

EP 0 173 177 B1

GENE, Vol. 18, No. 1, April 1982 (Amsterdam) P.J. GREENAWAY et al. "Human Cytomegalovirus DNA: Bam HI, Eco RI and Pst I restriction eudonuclease cleavage maps" Seiten 355-360

CELL, Vol. 36, No. 4, April 1984 (Cambridge, Mass.) F. WEBER et al. "An SV40 "Enhancer Trap" Incorporates Exogenous Enhancers of Generates Enhancers from its Own Sequences" Seiten 983-992

(74) Vertreter: **Becker, Heinrich Karl Engelbert, Dr. et al HOECHST AKTIENGESELLSCHAFT Central Patent Department P.O. Box 80 03 20 W-6230 Frankfurt am Main 80(DE)**

## Beschreibung

Die Erfindung ist in den Ansprüchen 1 bis 3 definiert, bevorzugte Ausgestaltungen des Verfahrens nach Anspruch 3 sind in den Ansprüchen 4 und 5 wiedergegeben. Weitere vorteilhafte Ausgestaltungen folgen.

Die "Enhancer-Falle" ist beschrieben bei F. Weber et al., Cell 36 (1984) 983-992, zur HCMV-DNA siehe G. Jahn et al., J. Virology, Feb. 1984, Vol. 49, 363-370 und dort zitierte Literatur, ferner D. R. Thomsen et al., Proc. Natl. Acad. Sci. USA, 81 (1984), 659-663, und P. J. Greenaway et al., Gene 18 (1982) 355-360.

Der Enhancer ist in der HCMV-DNA im Hind III E-Fragment lokalisiert (Greenaway et al., a.a.O.), das das Pst I m-Fragment umfaßt (etwa 2,1 kb).

Nach Anspruch 2 wurden 2 Recombinanten isoliert, die 341 bzw. 262 bp HCMV-DNA enthielten, angeordnet bei Position -118 bis -458 bzw. -263 bis -524 der publizierten DNA-Sequenz (Greenaway et al., a.a.O.). Die Überlappung von 196 bp enthält einen wesentlichen Teil des Enhancers. Deletionsmutanten, z. B. erhalten durch Aha II und Religieren der Fragmente in unterschiedlichen Kombinationen, sind ebenfalls Enhancer-aktiv.

DNA, die zu mindestens 75, vorzugsweise mindestens 80 % Sequenz-homolog mit reisolierter HCMV-spezifischer Enhancer-DNA ist bzw. damit hybridisiert, ist ebenfalls Erfindungsgegenstand.

Der Enhancer erhöht die Expression von Kaninchen-Betaglobin in HeLa-Zellen nach Einbau unterhalb des entsprechenden Gens um mindestens zwei Größenordnungen, unabhängig von der Orientierung. Damit ist der Enhancer um den Faktor 3-5 besser als der von SV40, abhängig vom Wirtssystem.

Der HCMV-Enhancer besitzt die Aktivität in einem weiten Spektrum an Wirtszellen (Primaten-, Mäuse-, Ratten- und Froschzellen). Er stimuliert die Expression von Proteinen in eukaryotischen Systemen und erleichtert dadurch die Produktion von modifizierten Proteinen, z. B. Glycoproteinen.

Bei der Anwendung kann auch der HCMV-eigene Promotor eliminiert werden, beispielsweise durch Abbau von etwa 100 bp mit Bal 31 über die Sac I-Schnittstelle hinaus. Gegebenenfalls kann die Enhancer-Sequenz durch Anbau von Adaptoren oder Linkern modifiziert werden.

Bei Verwendung mit dem eigenen Promotor kann ein eukaryotischer Promotor ersetzt werden, z. B. durch Einbau inklusive der ersten "splice donor consensus sequence" des IE-Gens vor der "splice acceptor sequence" des zu exprimierenden Gens.

In dem folgenden Beispiel wird die Erfindung näher erläutert.

## Beispiel

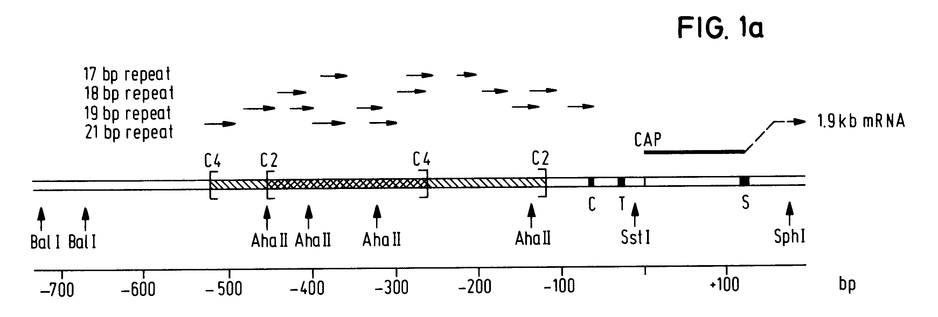

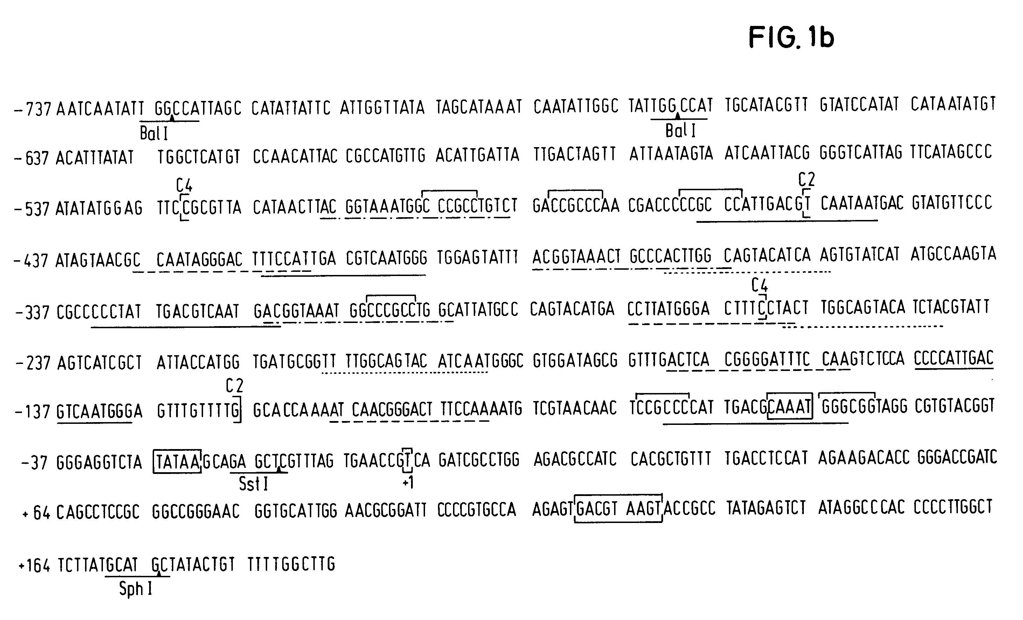

Nach Weber et al., a.a.O., wurde eine "Enhancer-Falle" durch Entfernen der 72 bp "repeat region" (Schneiden mit XbaI und KpnI) aus dem SV40-Genom hergestellt. Das PstI-m-Fragment (2,1 kb) von HCMV, Stamm AD 169, wurde durch Sonifikation in etwa 300 bp-große Stücke zerlegt und mit der "Enhancer-Falle" cotransfiziert. Aus den Kolonien, die das beste lytische Wachstum zeigten, wurde die rekombinante DNA isoliert. Es wurde durch Sequenzierung ein 262 bp-Segment von HCMV-DNA ermittelt, wobei auf einer Seite eine Ende-an-Ende-Ligierung eingetreten war, während auf der anderen Seite die Rekombination über eine 6 bp-Homologie zwischen HCMV (Nucleotide -531 bis -526, Figur 1a) und SV40 (Nucleotide 67 bis 72) erfolgte. Hierdurch ergab sich eine Deletion von 27 bp der SV40-DNA (Nucleotide 73 bis 99), die beide 21 bp-"repeats" des SV40 "early promoter" betraf. In der Restriktionskarte (Figur 1a) und in der DNA-Sequenz (Figur 1b) ist das 262 bp-Segment durch die eckigen Klammern und die Angabe "C4" bezeichnet. Eine weitere Enhancer-aktive Rekombinante mit 341 bp HCMV-DNA erwies sich als Ligationsprodukt mit den Enden eines linearen "Enhancer-Falle"-Moleküls (wobei einige Basen von den KpnI- und XbaI-Enden der SV40-DNA abgespalten waren, vermutlich durch exonucleolytischen Abbau vor der Ligation innerhalb der transfizierten Zelle). Die HCMV-DNA dieser Rekombinante ist in den Figuren 1a und 1b mit "C2" bezeichnet; sie erstreckt sich von -188 bis -458. Die Segmente C2 und C4 überlappen sich somit über einen Bereich von 196 bp.

Das Hind III C-Fragment des rekombinanten Viruses mit der Insertion C4 und das PstI-m-Fragment von HCMV wurden zunächst in pUC8 (J. Vieira et al., Gene 19 (1982) 259-268) in beiden Orientierungen kloniert, als Hind III-SalI-Fragmente ausgeschnitten und zwischen die HindIII- und XhoI-Schnittstelle von pβX14 rekloniert, also unterhalb des Kaninchen-β-Globin-Gens (J. Banerji et al., Cell 27 (1981) 299-308; J. de Villiers et al., Nucl. Acids Res. 9 (1981) 6251-6254; S. Rusconi et al., Proc. Natl. Acad. Sci. USA 78 (1981) 5051-5055; H. Weber et al., ICN-UCLA Symp. Mol. Cell. Biol. 33 (1981) 367; B. Wasylyk et al., Cell 32 (1983) 503-514). Die Enhancer-Wirkung auf die β-Globin-Transkription wurde durch S1 Nuclease-Analyse cytoplasmatischer RNA nach "transient expression" in HeLa-Zellen bestimmt. Alle Konstruktionen wurden zusammen mit analogen Konstruktionen mit dem SV40-Enhancer unter standardisierten Bedingungen verglichen. Es zeigte sich, daß der HCMV-Enhancer die Synthese von β-Globin mindestens um 2 Größenordnungen - unabhängig von der Orientierung - steigert.

**Patentansprüche**
**Patentansprüche für folgende Vertragsstaaten
: BE, CH, DE, FR, GB, IT, LI, LU, NL, SE**

1. DNA-Fragment mit Enhancer-Aktivität für euka-ryotische Expressionssysteme, dadurch ge-kennzeichnet, daß sich das DNA-Fragment von der PstI-Schnittstelle, welche stromaufwärts vom Transkriptionsstartpunkt des PstI-m-Fragments des Human-Cytomegalievirus (HCMV) liegt, bis zur Position - 118 des genannten Fragments erstreckt, oder Enhancer-aktive Teile oder Mutanten davon.

2. DNA-Fragment nach Anspruch 1, dadurch gekennzeichnet, daß sich das DNA-Fragment von den Positionen - 524 bis - 118, - 524 bis - 263, - 524 bis - 458, - 458 bis - 118, - 458 bis - 263 oder - 263 bis - 118, vorzugsweise von - 118 bis - 458 oder - 263 bis - 524, erstreckt.

3. Verfahren zur Verbesserung eukaryotischer Expressionssysteme, dadurch gekennzeichnet, daß oberhalb oder unterhalb des Strukturgens bzw. der Regulationsregion ein DNA-Fragment nach Anspruch 1 oder 2 eingebaut wird.

4. Verfahren nach Anspruch 3, dadurch gekennzeichnet, daß das DNA-Fragment höchstens etwa 7000 bp oberhalb oder unterhalb der genannten Stellen eingebaut wird.

5. Verfahren nach Anspruch 3 oder 4, dadurch gekennzeichnet, daß das DNA-Fragment weniger als 3000 bp oberhalb oder unterhalb der genannten Stellen eingebaut wird.

6. Verwendung eines DNA-Fragments nach Anspruch 1 oder 2 zur Verbesserung eukaryoti-scher Expressionssysteme, dadurch gekenn-zeichnet, daß das DNA-Fragment oberhalb oder unterhalb des Strukturgens bzw. der Re-gulationsregion eingebaut wird.

**Patentansprüche für folgenden Vertragsstaat :
AT**

1. Verfahren zur Herstellung eines DNA-Fragments mit Enhancer-Aktivität für eukaryotische Expressionssysteme, dadurch gekennzeichnet, daß ein DNA-Fragment, das sich von der PstI-Schnittstelle, welche stromaufwärts vom Transkriptionsstartpunkt des PstI-m-Fragments des Human-Cytomegalievirus (HCMV) liegt, bis zur Position - 118 des genannten Fragments erstreckt, oder Enhancer-aktive Teile oder Mutanten davon isoliert wird.

2. Verfahren zur Herstellung eines DNA-Fragments nach Anspruch 1, dadurch gekennzeichnet, daß sich das DNA-Fragment von den Positionen - 524 bis - 118, - 524 bis - 263, - 524 bis - 458, - 458 bis - 118, - 458 bis - 263 oder - 263 bis - 118, vorzugsweise von - 118 bis - 458 oder - 263 bis - 524, erstreckt.

3. Verfahren zur Verbesserung eukaryotischer Expressionssysteme, dadurch gekennzeichnet, daß oberhalb oder unterhalb des Strukturgens bzw. der Regulationsregion ein DNA-Fragment, das sich von der PstI-Schnittstelle, welche stromaufwärts vom Transkriptionsstartpunkt des PstI-m-Fragments des Human-Cytomegalievirus (HCMV) liegt, bis zur Posititon - 118 des genannten Fragments erstreckt, oder Enhancer-aktive Teile oder Mutanten davon eingebaut wird.

4. Verfahren zur Verbesserung eukaryotischer Expressionssysteme nach Anspruch 3, dadurch gekennzeichnet, daß oberhalb oder unterhalb des Strukturgens bzw. der Regulationsregion ein DNA-Fragment, das sich von den Positionen - 524 bis - 118, - 524 bis - 263, - 524 bis - 458, - 458 bis - 118, - 458 bis - 263 oder - 263 bis - 118, vorzugsweise von - 118 bis - 458 oder - 263 bis - 524 erstreckt, eingebaut wird.

5. Verfahren nach Anspruch 3 oder 4, dadurch gekennzeichnet, daß das DNA-Fragment höchstens etwa 7000 bp oberhalb oder unterhalb der genannten Stellen eingebaut wird.

6. Verfahren nach mindestens einem der Ansprüche 3 bis 5, dadurch gekennzeichnet, daß das DNA-Fragment weniger als 3000 bp oberhalb oder unterhalb der genannten Stellen eingebaut wird.

7. Verwendung eines DNA-Fragments, das sich von der PstI-Schnittstelle, welche stromaufwärts vom Transkriptionsstartpunkt des PstI-m-Fragments des Human-Cytomegalievirus (HCMV) liegt, bis zur Position - 118 des genannten Fragments erstreckt, oder Enhancer-aktive Teile oder Mutanten davon, zur Verbesserung eukaryotischer Expressionssysteme, dadurch gekennzeichnet, daß das DNA-Fragment oberhalb oder unterhalb des Strukturgens bzw. der Regulationsregion eingebaut wird.

8. Verwendung eines DNA-Fragments nach Anspruch 7, das sich von den Positionen - 524 bis - 118, - 524 bis - 263, - 524 bis - 458, - 458 bis - 118, - 458 bis - 263 oder - 263 bis - 118, vorzugsweise von - 118 bis - 458 oder - 263 bis - 524 erstreckt.

## Claims

**Claims for the following Contracting States : BE, CH, DE, FR, GB, IT, LI, LU, NL, SE**

1. A DNA fragment with enhancer activity for eukaryotic expression systems, wherein the DNA fragment extends from the PstI cleavage site which is located upstream from the transcription start point of the PstI-m fragment of human cytomegalovirus (HCMV) to position -118 of said fragment, or enhancer-active parts or mutants thereof.

2. A DNA fragment as claimed in claim 1, wherein the DNA fragment extends from positions -524 to -118, -524 to -263, -524 to -458, -458 to -118, -458 to -263 or -263 to -118, preferably from -118 to -458 or -263 to -524.

3. A process for the improvement of eukaryotic expression systems, which comprises incorporation of a DNA fragment as claimed in claim 1 or 2, upstream or downstream of the structural gene or of the regulation region.

4. The process as claimed in claim 3, wherein the DNA fragment is incorporated not more than about 7,000 bp upstream or downstream of the sites specified.

5. The process as claimed in claim 3 or 4, wherein the DNA fragment is incorporated less than 3,000 bp upstream or downstream of the sites specified.

6. The use of a DNA fragment as claimed in claim 1 or 2 for the improvement of eukaryotic expression systems, wherein the DNA fragment is incorporated upstream or downstream of the structural gene or of the regulation region.

**Claims for the following Contracting State : AT**

1. A process for the preparation of a DNA fragment with enhancer activity for eukaryotic expression systems, which comprises isolation of a DNA fragment which extends from the PstI cleavage site which is located upstream from the transcription start point of the PstI-m fragment of human cytomegalovirus (HCMV) to position -118 of said fragment, or enhancer-active parts or mutants thereof.

2. The process for the preparation of a DNA fragment as claimed in claim 1, wherein the DNA fragment extends from positions -524 to -118, -524 to -263, -524 to -458, -458 to -118, -458 to -263 or -263 to -118, preferably from -118 to -458 or -263 to -524.

3. A process for the improvement of eukaryotic expression systems, which comprises incorporation of a DNA fragment which extends from the PstI cleavage site which is located upstream from the transcription start point of the PstI-m fragment of human cytomegalovirus (HCMV) to position -118 of said fragment, or enhancer-active parts or mutants thereof, upstream or downstream of the structural gene or of the regulation region.

4. The process for the improvement of eukaryotic expression systems as claimed in claim 3, wherein a DNA fragment which extends from positions -524 to -118, -524 to -263, -524 to -458, -458 to -118, -458 to -263 or -263 to -118, preferably from -118 to -458 or -263 to -524 is incorporated upstream or downstream of the structural gene or of the regulation region.

5. The process as claimed in claim 3 or 4, wherein the DNA fragment is incorporated not more than about 7,000 bp upstream or downstream of the sites specified.

6. The process as claimed in at least one of claims 3 to 5, wherein the DNA fragment is incorporated less than 3,000 bp upstream or downstream of the sites specified.

7. The use of a DNA fragment which extends from the PstI cleavage site which is located upstream from the transcription start point of the PstI-m fragment of human cytomegalovirus (HCMV) to position -118 of said fragment, or enhancer-active parts or mutants thereof for the improvement of eukaryotic expression systems, wherein the DNA fragment is incorporated upstream or downstream of the structural gene or of the regulation region.

8. The use of a DNA fragment as claimed in claim 7, which extends from positions -524 to -118, -524 to -263, -524 to -458, -458 to -118, -458 to -263 or -263 to -118, preferably from -118 to -458 or -263 to -524.

## Revendications

**Revendications pour les Etats contractants suivants : BE, CH, DE, FR, GB, IT, LI, LU, NL, SE**

1. Fragment d'ADN possèdant une activité d'activateur pour des systèmes d'expression eucaryotes, caractérisé en ce que le fragment d'ADN s'étend du site de coupure de PstI, qui se trouve en amont du point de départ de la transcription du fragment PstI-m du cytomégalovirus humain (HCMV), jusqu'à la position - 118 du fragment indiqué, ou parties ou mutants de celui-ci ayant une activité d'activateur.

2. Fragment d'ADN selon la revendication 1, caractérisé en ce que le fragment d'ADN va des positions - 524 à - 118, - 524 à - 263, - 524 à - 458, - 458 à - 118, - 458 à - 263 ou - 263 à - 118, de préférence de - 118 à - 458 ou de - 263 à - 524.

3. Procédé pour améliorer des systèmes d'expression eucaryotes, caractérisé en ce qu'on insère un fragment d'ADN selon la revendication 1 ou 2, en amont ou en aval du gène de structure ou de la région régulatrice.

4. Procédé selon la revendication 3, caractérisé en ce qu'on insère le fragment d'ADN, au plus, à environ 7000 bp en amont ou en aval de la position mentionnée.

5. Procédé selon la revendication 3 ou 4, caractérisé en ce qu'on insère le fragment d'ADN à moins de 3000 bp en amont ou en aval de la position mentionnée.

6. Utilisation d'un fragment d'ADN selon la revendication 1 ou 2, pour améliorer des systèmes d'expression eucaryotes, caractérisée en ce qu'on insère le fragment d'ADN en amont ou en aval du gène de structure ou de la région régulatrice.

**Revendications pour l'Etat contractant suivant : AT**

1. Procédé pour préparer un fragment d'ADN possèdant une activité d'activateur pour des systèmes d'expression eucaryotes, caractérisé en ce qu'on isole un fragment d'ADN qui s'étend du site de coupure de PstI, qui se trouve en amont du point de départ de la transcription du fragment PstI-m du cytomégalovirus humain (HCMV), jusqu'à la position - 118 du fragment cité, ou des parties ou mutants de celui-ci ayant une activité d'activateur.

2. Procédé pour préparer un fragment d'ADN selon la revendication 1, caractérisé en ce que le fragment d'ADN va des positions - 524 à - 118, - 524 à - 263, - 524 à - 458, - 458 à - 118, - 458 à - 263 ou - 263 à - 118, de préférence de - 118 à - 458 ou de - 263 à - 524.

3. Procédé pour améliorer des systèmes d'expression eucaryotes, caractérisé en ce qu'on insère, en amont ou en aval du gène de structure ou de la région régulatrice, un fragment d'ADN qui s'étend du site de coupure de PstI, qui se trouve en amont du point de départ de la transcription du fragment PstI-m du cytomégalovirus humain (HCMV), jusqu'à la position - 118 du fragment cité, ou des parties ou mutants de celui-ci avant une activité d'activateur.

4. Procédé pour améliorer des systèmes d'expression eucaryotes selon la revendication 3, caractérisé en ce qu'on insère, en amont ou en aval du gène de structure ou de la région régulatrice, un fragment d'ADN qui va des positions - 524 à - 118, - 524 à - 263, - 524 à - 458, - 458 à - 118, - 458 à - 263 ou - 263 à - 118, de préférence de - 118 à - 458 ou de - 263 à - 524.

5. Procédé selon la revendication 3 ou 4, caractérisé en ce qu'on insère le fragment d'ADN, au plus, à environ 7000 bp en amont ou en aval de la position mentionnée.

6. Procédé selon au moins une des revndications 3 à 5, caractérisé en ce qu'on insère le fragment d'ADN à moins de 3000 bp en amont ou en aval de la position mentionnée.

7. Utilisation d'un fragment d'ADN qui s'étend du site de coupure de PstI, qui se trouve en amont du point de départ de la transcription du fragment PstI-m du cytomégalovirus humain (HCMV), jusqu'à la position - 118 du fragment cité, ou de parties ou mutants de celui-ci ayant une activité d'activateur, pour améliorer des systèmes d'expression eucaryotes, caractérisée en ce qu'on insère le fragment d'ADN en amont ou en aval du gène de structure ou de la région régulatrice.

8. Utilisation, selon la revendication 7, d'un fragment d'ADN qui va des positions - 524 à - 118, - 524 à - 263, - 524 à - 458, - 458 à - 118, - 458 à - 263 ou - 263 à - 118, de préférence de - 118 à - 458 ou de - 263 à - 524.

FIG. 1a

# FIG. 1b

-737 AATCAATATT GGCCATTAGC CATATTATTC ATTGGTTATA TAGCATAAAT CAATATTGGC TATTGGCCAT TGCATACGTT GTATCCATAT CATAATATGT
         BalI                                                                    BalI

-637 ACATTTATAT TGGCTCATGT CCAACATTAC CGCCATGTTG ACATTGATTA TTGACTAGTT ATTAATAGTA ATCAATTACG GGGTCATTAG TTCATAGCCC

-537 ATATATGGAG TTCCGCGTTA CATAACTTAC GGTAAATGGC CCGCCTGTCT GACCGCCCAA CGACCCCCGC CCATTGACGT CAATAATGAC GTATGTTCCC
                  C4                                                                                C2

-437 ATAGTAACGC CAATAGGGAC TTTCCATTGA CGTCAATGGG TGGAGTATTT ACGGTAAACT GCCCACTTGG CAGTACATCA AGTGTATCAT ATGCCAAGTA
                                                                                        C4

-337 CGCCCCCTAT TGACGTCAAT GACGGTAAAT GGCCCGCCTG GCATTATGCC CAGTACATGA CCTTATGGGA CTTTCCTACT TGGCAGTACA TCTACGTATT

-237 AGTCATCGCT ATTACCATGG TGATGCGGTT TTGGCAGTAC ATCAATGGGC GTGGATAGCG GTTTGACTCA CGGGGATTTC CAAGTCTCCA CCCCATTGAC
                  C2

-137 GTCAATGGGA GTTTGTTTTG GCACCAAAAT CAACGGGACT TTCCAAAATG TCGTAACAAC TCCGCCCCAT TGACGCAAAT GGGCGGTAGG CGTGTACGGT

-37 GGGAGGTCTA TATAAGCAGA GCTCGTTTAG TGAACCGTCA GATCGCCTGG AGACGCCATC CACGCTGTTT TGACCTCCAT AGAAGACACC GGGACCGATC
                   SstI                     +1

+64 CAGCCTCCGC GGCCGGGAAC GGTGCATTGG AACGCGGATT CCCCGTGCCA AGAGTGACGT AAGTACCGCC TATAGAGTCT ATAGGCCCAC CCCCTTGGCT

+164 TCTTATGCAT GCTATACTGT TTTTGGCTTG
          SphI